# EUROPEAN PATENT APPLICATION

(11) **EP 4 321 890 A1**
(43) Date of publication of application: **14.02.2024**
(21) Application number: 22189428.0
(22) Date of filing: 09.08.2022
(51) Int. Cl.: G01R 33/56, G06T 7/00, G01R 33/50, G01R 33/561, G06N 3/02, A61B 5/055, G06T 5/00, A61B 5/00

(54) **RECONSTRUCTION PARAMETER DETERMINATION FOR THE RECONSTRUCTION OF SYNTHESIZED MAGNETIC RESONANCE IMAGES**

(71) Applicant: Koninklijke Philips N.V., 5656 AG Eindhoven (NL)
(72) Inventor: WENZEL, Fabian, Eindhoven (NL); FLÄSCHNER, Nick, Eindhoven (NL); EWALD, Arne, 5656AG Eindhoven (NL); VAN DER VEN, John, 5656AG Eindhoven (NL)
(74) Representative: Philips Intellectual Property & Standards

(57) **Abstract**

Disclosed herein is a medical system (100, 300) comprising a memory (110) storing machine executable instructions (120) and an anatomical detection module (122), and a computational system (104). The execution of the machine executable instructions causes the computational system to: receive (200) a set of magnetic resonance images (124) descriptive of a field of view (109) of a subject (318) acquired according to a synthetic magnetic resonance imaging protocol; receive (202) an anomaly indicator (126) from the anomaly detection module in response to inputting at least one of the set of magnetic resonance images into the anomaly detection module; determine (204) a set of reconstruction parameters (128) using the anomaly indicator; and reconstruct (206) a synthesized magnetic resonance image (134) from the set of magnetic resonance images and the set of reconstruction parameters.

## Description

### FIELD OF THE INVENTION

The invention relates to magnetic resonance imaging, in particular to synthesized or synthetic magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially and imaged using MRI. A difficulty with performing magnetic resonance imaging is that it takes time to acquire the k-space data necessary to reconstruct the magnetic resonance imaging. When the k-space data is acquired different imaging protocols can be used to weight the image differently. For example, a magnetic resonance image could be weighted to show proton density, the spatial variation of T1, or the spatial variation of T2 or T2-star. These different weightings are commonly referred to as "contrasts" or "contrast types." Physicians and other medical professionals are accustomed to viewing particular "contrasts." Other imaging techniques have been developed in recent years such as magnetic resonance imaging fingerprinting (MRF) or Synthetic Magnetic Resonance Imaging (SyntAC or 3D-QALAS). In these techniques, k-space data is used to acquire images that may be used to construct synthetic or mapped images that mimic or reproduce typical or canonical "contrasts."

In synthetic magnetic resonance imaging, a set of magnetic resonance images descriptive of a field of view depicting a subject are received. This set of magnetic resonance images represents essentially a standardized acquisition (for a particular synthetic MR reconstruction) that provides the data which may be used to calculate spatially varying quantitative maps descriptive of the subject. These quantitative maps then provide enough information to simulate other magnetic resonance images using an MR signal equation. Two techniques which enable this are Magnetic Resonance Fingerprinting (MRF) and Synthetic Magnetic Resonance Imaging (SyntAc or 3D-QALAS).

United States patent application publication US2021174937A1 discloses a diagnostic machine for a medical diagnosis of raw medical imaging data generated by a medical imaging machine as opposed to a medical diagnosis of a medical image conventionally reconstructed from the raw medical imaging data. In operation, the raw diagnostic engine includes a medical imaging diagnostic controller implementing a dimension reduction pre-processor for selecting or extracting one or more dimension reduced feature vectors from the raw medical imaging data, and further implementing a raw diagnostic artificial intelligence engine for rendering a diagnostic assessment of the raw medical imaging data as represented by the dimension reduced feature vector(s). The medical imaging diagnostic controller may further control a communication of the diagnostic assessment of the raw medical imaging data (e.g., a display, a printing, an emailing, a texting, etc.).

The publication Baur, et. al., "Autoencoders for Unsupervised Anomaly Segmentation in Brain MR Images A Comparative Study," arXiv 2004.03271, https://doi.org/10.48550/arXiv.2004.03271 discloses the detailed use of variational autoencoders to segment anomalies in MRI brain images.

### SUMMARY OF THE INVENTION

The invention provides for a medical system, a computer program, and a method in the independent claims. Embodiments are given in the dependent claims.

In various synthesized or synthetic magnetic resonance imaging techniques one may typically reconstruct synthesized magnetic resonance images of various types that are modeled after real magnetic resonance imaging acquisitions. This includes the ability to model the choice of various reconstruction parameters such as the echo time (TR) or repetition time (TR) that are set at the time of acquiring the k-space data or even the pulse sequence type used to acquire the k-space data. The choice of these parameters affects the image contrast within the image and ultimately what types of anatomical structures are visible within the synthesized magnetic resonance image.

To make an anomaly visible or optimally visible in a synthesized magnetic resonance image may therefore involve trial and error to set reconstruction parameters correctly. Anomalies may encompass anatomical anomalies or abnormal tissue pathologies or abnormal tissue structures or properties such as white matter hyper-intensities. Embodiments may provide a means of automatically determining the set of reconstruction parameters for a synthesized magnetic resonance image automatically. This may be achieved by using an anomaly detection module that is configured to receive the set of magnetic resonance images (or a subset of these images) and to output an anomaly indicator. This anomaly indicator is then used to determine the set of reconstruction parameters which are optimized or chosen to display the detected anomaly in a reconstructed synthetic magnetic resonance image.

The concept of generating synthetic contrasts interactively or by supplying a set of parameters for specific maps to the Radiologist to inspect several images for the assessment and identification of pathologies, resulting in a time-consuming workflow.

Examples may have the benefit of speeding up the workflow for reading images in the context of Synthetic MRI by anomaly detection on the quantitative sequences and based on its result, choosing a predefined standard synthetic sequence or dedicated parameters for a synthetic contrast, such that pathologies can be visualized in an optimal manner allowing fast visual assessment and, therefore, enhancing the workflow of the Radiologist.

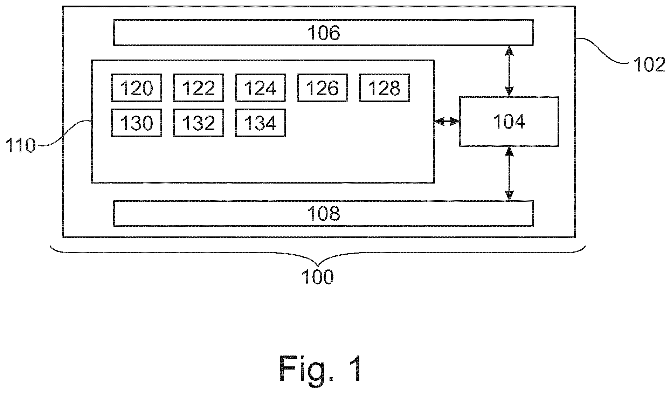

Examples may contain one or more of the following subsequent work steps, which are described in more detail:
1. Anomaly detection: AI-based anomaly detection using quantitative SyntAc images can be done with different approaches: (i) Anatomy-driven anomaly detection, in which symmetrified contrast between the left and right hemisphere is analyzed, or other known approaches, such as variational autoencoders. For SyntAc in particular, anomalies per voxel might additionally be derived by deviations from normal tissue properties in the brain as it can be seen in the lower left part of Fig. 1. All approaches result in a map indicating voxel-wise abnormality and might be post-processed by filtering techniques in order to remove artifacts in detected anomalies, such as removal of individual abnormal voxels. For this optional step, standard image processing algorithms like thresholding, smoothing or filtering might be applied.
2. Derivation of optimal sequence parameters for synthetic contrast generation. Based on the results of step 1, parameters for generating synthetic contrast (TE, TR, and TI) are determined such that the range of gray values in the area in which anomalies are suspected, is maximized, leading to a pronounced presentation of abnormalities.
3. As an option, these parameters might be optimized not for the complete volume, but for each suspected pathology in the image individually. A final synthetic image can then be merged via interpolation such that the presentation of each suspected pathology will be optimal.

In one aspect the invention provides for a medical system that comprises a memory storing machine-executable instructions and an anatomical detection module. The medical system further comprises a computational system. Execution of the machine-executable instructions causes the computational system to receive a set of magnetic resonance images descriptive of a field of view of the subject acquired according to a synthetic magnetic resonance imaging protocol. The synthetic magnetic resonance imaging protocol may specify the acquisition parameters used for acquiring the set of magnetic resonance images and/or the reconstruction technique used in reconstructing the set of magnetic resonance images.

Execution of the machine-executable instructions further causes the computational system to receive an anomaly indicator from an anomaly detection module in response to inputting at least one of the set of magnetic resonance images into the anomaly detection module. The anomaly indicator may take different forms in different examples. In one case the anomaly indicator may simply indicate the presence of an anomaly within the set of magnetic resonance images as a label. For example, it may indicate that the set of magnetic resonance images contains a tumor of a particular type or anomalous tissue structure.

In this case, the anomaly indicator may not provide an indication of where this anomaly is located within the field of view. In other instances, the anomaly indicator may provide a location or bounding box within the field of view to indicate where an anomaly is located. In yet other examples the anomaly indicator may provide an indicator or bounding box for a location of an anomaly as well as a label to the type or identification of the anomaly type.

Execution of the machine-executable instructions further causes the computational system to determine a set of reconstruction parameters using the anomaly indicator. The set of reconstruction parameters used herein are instructions or details which are used for the reconstruction of a synthetic magnetic resonance image from the set of magnetic resonance images. For example, in the well-known SyntAC or 3D-QALAS technique synthetic contrast-weighted images are created using known T1, T2, and proton density values. The reconstruction parameters in this case may for example be the TE and TR times. This may for example depend upon the various model used for reconstructing the synthetic magnetic resonance image.

However, the set of reconstruction parameters may be chosen such that a synthesized magnetic resonance image has the anomaly emphasized or displayed in a way which is readily accessible. The determination of the set of reconstruction parameters may be done in a variety of ways. For example, if the anomaly indicator contains an identification of the type of anomaly then the set of reconstruction parameters could simply be recalled from a lookup table or a database. In other examples, if the anomaly indicator indicates a location then the reconstruction of the synthesized magnetic resonance image can be performed in an iterative fashion so that within the identified region where the anomaly is located various image parameters such as a contrast distribution can be made to match a predetermined range or set of values. That is to say the determination of the set of reconstruction parameters may for example be implemented by the machine-executable instructions.

Execution of the machine-executable instructions further causes the computational system to reconstruct a synthesized magnetic resonance image from the set of magnetic resonance images and the set of reconstruction parameters. This embodiment may be beneficial because the synthesized magnetic resonance image may have its reconstruction controlled or guided such that anomalies, which are detected by the anomaly detection module, may be displayed in a way which emphasizes or displays the structure of the anomaly. For example, the region in which an anomaly is contained may have its contrast maximized so that on a particular display or graphic type the structure of the anomaly is presented in detail.

In another embodiment the field of view is descriptive of a right hemisphere and a left hemisphere of a brain of a subject. The anomaly indicator comprises one or more anatomically anonymous locations in the field of view. The anomaly detection module is further configured to spatially locate the one or more anomaly in the brain of the subject using a symmetrified contrast between the right hemisphere and the left hemisphere of the brain of the subject. There is an expectation that the anatomy of the left hemisphere and right hemisphere of a brain of a subject may have similarities. An anomalous structure such as a tumor may cause a large asymmetry between the right hemisphere and the left hemisphere of the brain of the subject. The use of a symmetrified contrast between the right hemisphere and the left hemisphere of the brain may be used to identify such types of anomalies.

In another embodiment the anomaly indicator comprises one or more anatomically anomalous locations in the field of view. The anomaly detection module comprises an autoencoder neural network configured to output an autoencoded image for each of the at least one of the set of magnetic resonance images. Receiving an anomaly indicator in response to inputting the at least one of the set of magnetic resonance images into the anomaly detection module comprises receiving the autoencoded image in response to inputting the at least one set of magnetic resonance images into the autoencoder neural network. Receiving an anomaly indicator in response to inputting the at least one of the set of magnetic resonance images into the anomaly detection module further comprises determining the one or more anatomically anomalous locations by comparing the autoencoded image of each of the at least one of the set of magnetic resonance images with the at least one of the set of magnetic resonance images.

In this embodiment an autoencoder is used to produce an image in response to receiving at least one of the set of magnetic resonance images into the autoencoder network as input. The expectation of an autoencoder is that it takes an image and then produces an identical image. If an autoencoder is trained such that only images that are anatomically correct are used, the autoencoder may produce errors when it encounters anomalies. The comparison between the input images and the output images may therefore provide a means of easily identifying anatomically anomalous regions.

The autoencoder may be any autoencoder architecture which is able to take images as input and output an autoencoder version of the same images as output. There may be a multiple inputs and outputs for each image. Or a series of autoencoders, one for each of the set of images is used. The autoencoder may for example be a fully-connected autoencoder, a sparse autoencoder, a deep (fully-connected) autoencoder, a convolutional autoencoder, or a variational autoencoder.

The training data for these autoencoders may be sets of magnetic resonance images that do not contain anomalies. The images can be input into the autoencoder during training and the output can be compared to the input image.

In another embodiment execution of the machine-executable instructions further causes the computational system to iteratively vary the set of reconstruction parameters and reconstruct the synthesized magnetic resonance image to adjust the image contrast of the one or more anatomically anomalous locations to have a predetermined image contrast range. In this embodiment determination of the set of reconstruction parameters is performed at the same time as the reconstruction. Once the anomalous locations have been determined the predetermined image contrast range can be defined for these locations. The reconstruction can be started with a seed value of reconstruction parameters which is then varied until the obtained predetermined image contrast range within these regions is obtained.

In another embodiment the anomaly indicator indicates multiple anatomically anomalous locations. The synthesized magnetic resonance image is reconstructed for each of the multiple anatomically anomalous locations resulting in a set of synthesized magnetic resonance images. In this embodiment the anomaly indicator indicates or locates multiple regions of the field of view where there may be an anomaly. As there are multiple locations, there is a synthesized magnetic resonance image that is reconstructed for each of these locations. For example, an image could be made for each one of these which has the predetermined image contrast range.

In another embodiment execution of the machine-executable instructions further causes the computational system to construct a composite image from the set of synthesized magnetic resonance image locations by including the anatomically anomalous location from each of the set of synthesized magnetic resonance images and blending pixel values between the anatomically anomalous locations from each of the set of synthesized magnetic resonance images using the set of synthesized magnetic resonance images. It may be inconvenient to display the set of synthesized magnetic resonance images. In this embodiment, this set of synthesized magnetic resonance images is used to make a single composite image. The composite image is formed by including the anatomically anomalous location from each of these images that is to take the anomalous image from the field of view and paste them together into this composite image. Between these anatomically anomalous locations the pixel values are then blended. This for example may take different forms in different examples. The images which provide the multiple anatomically anomalous locations may have their values averaged or blended so that there is a smooth transition from one image contrast range to the next.

In another embodiment execution of the machine-executable instructions further causes the computational system to display the composite image on a graphical user interface. Execution of the machine-executable instructions further causes the computational system to receive a selection of an anatomically anomalous location within the composite image from the graphical user interface and then display the synthesized magnetic resonance image comprising the selected anatomically anomalous location. This embodiment provides a means of selecting one of the set of synthesized magnetic resonance images. The composite image that is displayed on the graphical user interface however may be different or unusual for a physician or other healthcare provider. They can then go and select the particular anatomically anomalous location, which is referred to herein as the selection of the anatomically anomalous location, and then this is used to select a particular synthesized magnetic resonance image from the set of synthesized magnetic resonance images. In this way, a physician or other healthcare provider can easily choose an image to review using the composite image.

In another embodiment the anatomical detection module comprises a convolutional neural network configured to output an anomaly classification as the anomaly indicator in response to receiving the at least one of the set of magnetic resonance images. In this embodiment instead of assigning a location a label is assigned to the set of magnetic resonance images. This for example may indicate a certain anomaly type or a certain tumor type. The knowledge of this particular anomaly type may be used to choose the set of reconstruction parameters then. For example, from prior experience or from previous reconstructions, it may be known what sort of set of reconstruction parameters may be used for a particular anomaly classification.

The convolutional neural network may use a neural network architecture that is configured to receive as input one or more images and to output a label. For example, a Deep Convolutional Neural Network (DCCN) architecture may be used. The convolutional neural network may be trained by inputting a set of neural networks that have been labeled with anomalies.

In another embodiment the anatomical detection module comprises a variational autoencoder neural network that has a latent space. The anatomical detection module is configured to output an anomaly classification as the anomaly indicator. The receiving of the anomaly indicator in response to inputting the at least one of the set of magnetic resonance images into the anomaly detection module comprises inputting the at least one of the set of magnetic resonance images into the variational autoencoder and then determining the anomaly classification using the out of distribution detection method on the latent space of the variational autoencoder.

Autoencoders use two neural network portions; the first encoding portion encodes the input image into a vector in a so-called latent space. The vector in the latent space is then input into the decoder portion of the autoencoder which then, if the variational autoencoder is properly trained, into an identical or very similar image. Variational autoencoders use additional constraints on the vector in latent space during training. Typically, there is a cost or penalty function which is assigned to the vector in latent space which forces it towards the center. This has the effect of forcing an order on the location of vectors in latent space. By monitoring the latent space during the use of the variational autoencoder it can be detected when the input image is outside of the training distribution.

A variational auto encoder which is configured to output an autoencoded image for each of the set of magnetic resonance images or a group of variational autoencoders, one for each of the set of magnetic resonance images, may be used. The variational audoencoder or encoders may be trained to reproduce magnetic resonance images without anomalies. Out of distribution techniques in the latent space of the variational autoencoder may then be used to detect anomalies.

In another embodiment the reconstruction parameters are determined by using the anomaly classification to search a reconstruction parameter lookup table or reconstruction parameter database. In these cases, the labels or the anomaly classification may be used to retrieve data from the reconstruction parameter lookup table or the reconstruction parameter database. The reconstruction parameter lookup table or reconstruction parameter database may be filled by using prior experience in reconstructing images to properly display a particular anomaly such as a tumor or other anatomical structure.

In another embodiment reconstructing the synthesized magnetic resonance image comprises determining a T1-dependent value (such as a T1 map), a T2-dependent value (such as a T2 map, T2-star map, an R2 map, or an R2-star map), and a proton density for each voxel of the field of view by performing a voxel wise state of a chosen signal intensity equation to the set of magnetic resonance images. Reconstructing the synthesized magnetic resonance image further comprises reconstructing a synthesized magnetic resonance image by calculating a signal intensity value for each voxel using a reconstruction signal intensity equation that takes the voxel wise T1-dependent value, the voxel wise T2-dependent value and the voxel wise proton density value and the reconstruction parameters as input.

In another embodiment the reconstruction parameters comprise an echo time, a repetition time, and preferably an inversion delay.

The above embodiments relating to constructing a synthesized magnetic resonance image from the T1-dependent value, a T2-dependent value, and a proton density may relate to synthetic magnetic resonance imaging or SyntAc. Synthetic MR offers standardized quantitative MR sequences (SyntAc) to be applied on magnetic resonance imaging systems. One of its claims is the derivation of standard contrasts like T1-weighted or T2-weighted images from SyntAc in a post-processing step. However, this is currently done interactively, and finding the best contrast for a specific pathology is time-consuming and not reproducible. This invention disclosure proposes to automatically find the best derived contrast by AI-based assessment of the quantitative SyntAc images such that the automatically suspected pathologies can be visually confirmed or rejected with very high confidence.

Automated detection of brain anomalies is a high-priority topic of the MR, aiming at the "speed" route towards the MR north star ("Less-than-5-min personalized exam for every patient, with fully automated workflow and decision support").

Synthetic MR offers standardized quantitative MR sequences (SyntAc) to be applied. One of its benefits may be the derivation of standard contrasts like T1-weighted or T2-weighted images from SyntAc in a post-processing step by freely choosing acquisition-related parameters such as TE, TR, and TI.

In another embodiment the set of magnetic resonance images forms a magnetic resonance fingerprint. The synthesized magnetic resonance images are reconstructed from the set of magnetic resonance images according to a magnetic resonance fingerprinting protocol.

During magnetic resonance fingerprinting a so-called dictionary or magnetic resonance fingerprinting dictionary is used to compare against a magnetic resonance fingerprint for each voxel. This enables the calculation of various spatially dependent values such as a T1 time, a T2 time or proton density. These values can be used to simulate a magnetic resonance signal and thereby recalculate a synthetic magnetic resonance image.

In another embodiment the medical system further comprises a magnetic resonance imaging system. The memory further contains pulse sequence commands configured to control the magnetic resonance imaging system to acquire k-space data according to the synthetic magnetic resonance imaging protocol. Execution of the machine-executable instructions further causes the computational system to acquire the k-space data by controlling the magnetic resonance imaging system with the pulse sequence commands and reconstruct the set of magnetic resonance images from the k-space data preferably according to the synthetic magnetic resonance imaging protocol.

In another aspect the invention provides for a method of operating a medical system. The method comprises receiving a set of magnetic resonance images descriptive of a field of view of a subject acquired according to a synthetic magnetic resonance imaging protocol. The method further comprises receiving an anomaly indicator from an anomaly detection module in response to inputting the at least one of the set of magnetic resonance images into the anomaly detection module. The method further comprises determining a set of reconstruction parameters using the anomaly indicator. The method further comprises reconstructing a synthesized magnetic resonance image from the set of magnetic resonance images and the set of reconstruction parameters.

In another aspect the invention provides for a computer program that comprises machine-executable instructions for execution by a computational system. The computer program further comprises an anatomical detection module for execution by the computational system.

Execution of the machine-executable instructions causes the computational system to receive a set of magnetic resonance images descriptive of a field of view of a subject acquired according to a synthetic magnetic resonance imaging protocol. Execution of the machine-executable instructions further causes the computational system to receive an anomaly indicator from an anomaly detection module in response to inputting at least one of the set of magnetic resonance images into the anomaly detection module. Execution of the machine-executable instructions further causes the computational system to determine a set of reconstruction parameters using the anomaly indicator. Execution of the machine-executable instructions further causes the computational system to reconstruct a synthesized magnetic resonance image from the set of magnetic resonance images and the set of reconstruction parameters.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system
Fig. 2 shows a flow chart which illustrates a method of using the medical system of Fig.1;
Fig. 3 illustrates a further example of a medical system

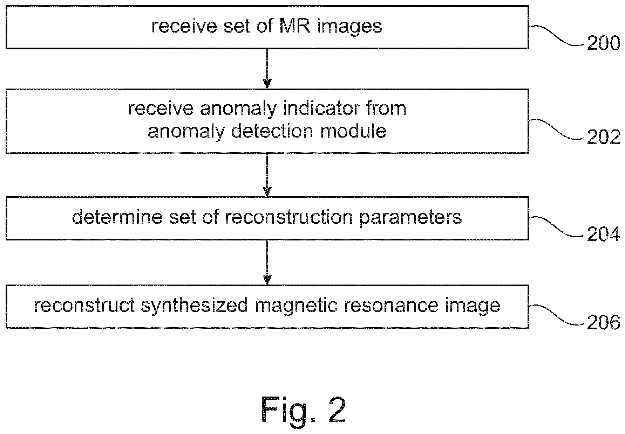

Fig. 2 shows a flow chart which illustrates a method of using the medical system of Fig.2;

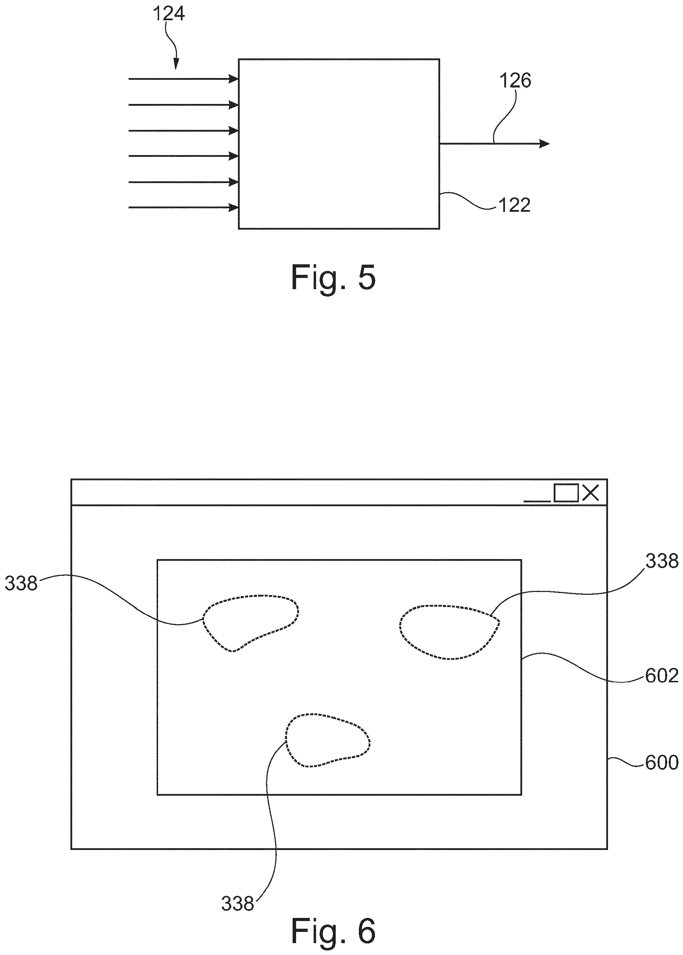

Fig. 5 illustrates an example of an anomaly detection module; and
Fig. 6 illustrates an example of a graphical user interface.

### DETAILED DESCRIPTION OF EMBODIMENTS

Like numbered elements in these figures are either equivalent elements or perform the same function. Elements which have been discussed previously will not necessarily be discussed in later figures if the function is equivalent.

Fig. 1 illustrates an example of a medical system 100. The medical system 100 is shown as comprising a computer 102. The computer 102 is intended to represent one or more computing devices at one or more locations. The computer comprises a computational system 104 that is likewise intended to represent one or more computational systems or computational cores that is located within one or more computer systems 102. The computational system 104 is shown as being in communication with an optional hardware interface 106, a user interface 108 and a memory 110. If other components are present the hardware interface 106 may enable the computational system 104 to exchange commands and data with those other components.

The hardware interface 106 therefore may enable the computational system 104 to control other components such as a magnetic resonance imaging system. The user interface 108, if it is present, may be used for providing a means to interact with and display data to an operator or user. Likewise, the medical system 100 may receive control commands from the user interface 108. The memory 110 is intended to represent various types of memory which are accessible to the computational system 104 and may for example be a non-transitory storage medium.

The memory 110 is shown as containing machine-executable instructions 120. The machine-executable instructions may enable the computational system 104 to perform such tasks as to control other components, to perform numerical tasks, and to perform basic image processing tasks. The memory 110 is further shown as containing an anatomical detection module 122 that is able to receive at least one of a set of magnetic resonance images 124 and in response output an anomaly indicator 126. The anatomical detection module 122 may for example be implemented using either a machine learning module or an algorithmic module, or a combination of the two. The memory 110 is further shown as containing the anomaly indicator 126 that was output by the anatomical detection module 122 in response to receiving at least one of a set of magnetic resonance images 124.

The memory 110 is shown as optionally containing a lookup table 130 or database 130. For example, given a particular anomaly indicator 126 may be used as a query for the lookup table or database 130; they may then output the set of reconstruction parameters 128. The memory 110 is further shown as containing a reconstruction algorithm 132 used to reconstruct a synthesized magnetic resonance image 134, which is also stored in the memory. In some instances, this reconstruction algorithm 132 may be iterative and may be used to generate the set of reconstruction parameters 128 at the same time as it reconstructs the synthesized magnetic resonance image 134. For example, the anomaly indicator 126 may represent a region within the field of view of the set of magnetic resonance images 124 which should have a predetermined contrast range. The reconstruction algorithm 132 could then iteratively change the reconstruction parameters 128 such that within this region the synthesized magnetic resonance image 134 has the predetermined image contrast range.

Fig. 2 shows a flowchart which illustrates a method of operating the medical system 100 of Fig. 1. First, in step 200, the set of magnetic resonance images 124 are received. These images 124 are descriptive of a field of view of a subject acquired according to a synthetic magnetic resonance imaging protocol. This may mean that the images which make up the set of magnetic resonance images are all for the same field of view and have different contrasts. A contrast for a magnetic resonance image implies a particular set of acquisition parameters and/or reconstruction parameters.

Next, in step 202, the anomaly indicator 126 is received from the anatomical detection module 122 in response to inputting at least one of the set of magnetic resonance images 124. Next, in step 204, the set of reconstruction parameters 128 is determined using the anomaly indicator 126 and this may for example be using the lookup table or database 130 as well as using an iterative reconstruction algorithm 132 as was described above.

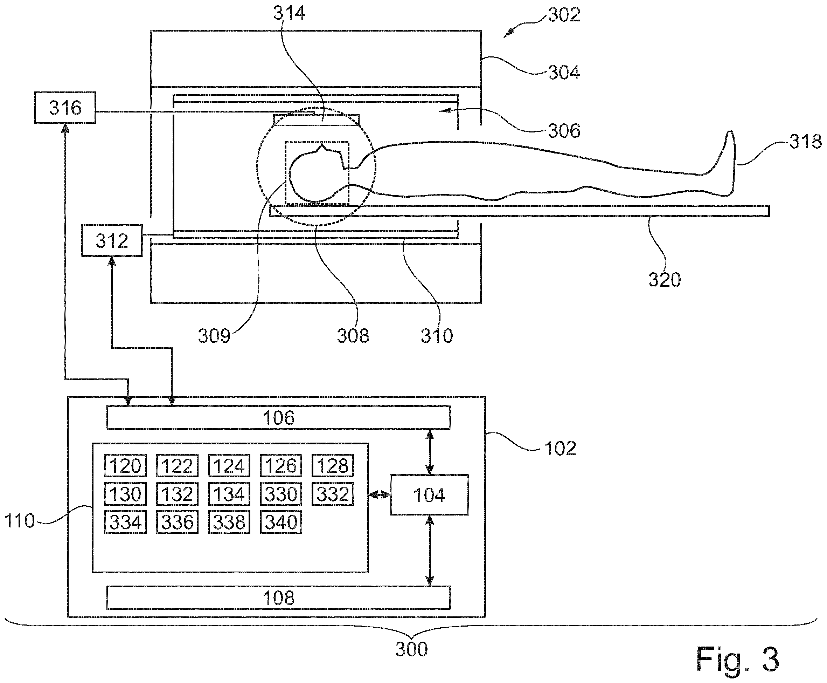

Fig. 3 illustrates a further example of a medical system 300. The medical system 300 depicted in Fig. 3 is similar to the medical system 100 of Fig. 1 except that it additionally comprises a magnetic resonance imaging system 302 that is controlled by the computational system 104.

The magnetic resonance imaging system 302 comprises a magnet 304. The magnet 304 is a superconducting cylindrical type of magnet with a bore 306 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory 110 is further shown as containing pulse sequence commands 330 that enable the computational system 104 to control the magnetic resonance imaging system 302 to acquire k-space data 332 according to the synthetic magnetic resonance imaging protocol. The pulse sequence commands 330 are commands or data which may be converted into commands which control the timing and function of various components of the magnetic resonance imaging system 302. The memory 110 is further shown as containing the k-space data 332 that has been acquired by controlling the magnetic resonance imaging system 302 using the pulse sequence commands 330. In this example, the set of reconstruction parameters 128 and the synthesized magnetic resonance image 134 are determined iteratively.

The memory 110 is further shown as containing a preliminary set of reconstruction parameters 334 that are used during reconstruction of a trial magnetic resonance image 336. Within the trial magnetic resonance image 336 is specified the anomalous location 338. Also within the memory 110 is a calculated image contrast range 340 within the anatomically anomalous location 338 of the trial magnetic resonance image 336. This is compared to a predetermined image contrast range 342. If the calculated image contrast range 340 differs from the predetermined image contrast range 342 the preliminary set of reconstruction parameters 334 can be modified and the trial magnetic resonance image 336 can be recalculated.

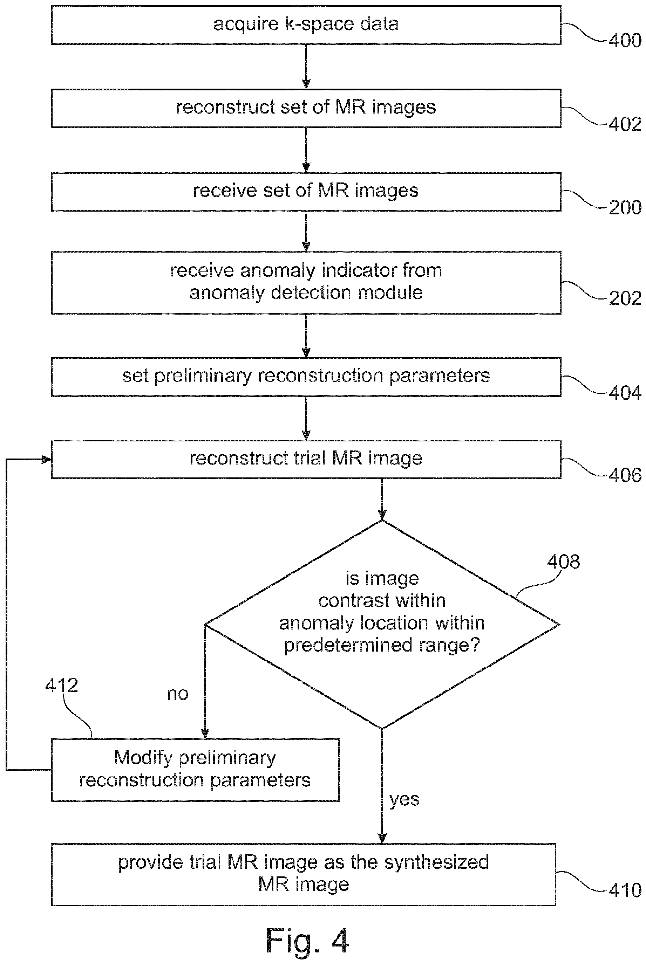

Fig. 4 shows a flowchart which illustrates a method of operating the medical system 300 of Fig. 3. First, in step 400, the k-space data 332 is acquired by controlling the magnetic resonance imaging system 302 with the pulse sequence commands 330. Next, in step 402, the set of magnetic resonance images 124 are reconstructed from the acquired k-space data 332. The method then proceeds to perform steps 200 and 202 as was illustrated in Fig. 2. Steps 404, 406, 408, 410, and 412 may be substituted for step 204 in Fig. 2. In this example, after step 202 is completed, the method then proceeds to step 404. In this case, the preliminary set of reconstruction parameters 334 is set or determined. Next, in step 406, the trial magnetic resonance image 336 is reconstructed from the set of magnetic resonance images 124 and the preliminary set of reconstruction parameters 334.

The method then proceeds to step 408, which is a question box and the question is 'is the calculated image contrast range 340 the same as the predetermined image contrast range 342'. If the answer is yes the method proceeds to step 410 and the trial magnetic resonance image 336 is provided as the synthesized magnetic resonance image 134. If the answer is no the method proceeds to step 412 where the set of preliminary reconstruction parameters 334 is modified and then the method proceeds back to step 406 where the trial magnetic resonance image 336 is recalculated. This loop then repeats until the calculated image contrast range 340 matches the predetermined image contrast range 342.

Fig. 5 illustrates an example of an anatomical detection module 122. The anatomical detection module 122 receives the set of magnetic resonance images 124 as input and then outputs the anomaly indicator 126. As was mentioned above, the anatomical detection module 122 may be implemented algorithmically, as a trained machine learning module, or a combination of the two.

Fig. 6 illustrates an example of a graphical user interface 600 that displays a composite image 602. The composite image in this example 602 is formed from a set of synthesized magnetic resonance images. Each has its own anomalous location 338. Within the composite image 602 the anomalous location 338 from each image is displayed and the space of the composite image 602 between these is formed by blending the individual synthesized magnetic resonance images. In this user interface if a user selects one of the anatomically anomalous locations 338 it then causes the image from which the anatomically anomalous location 338 was taken to be displayed. This graphical user interface 600 may therefore be used to rapidly display the various anatomically anomalous locations 338 and then to select a more clinically relevant image for the physician or healthcare provider to examine.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: anatomical detection module
- 124: set of magnetic resonance images
- 126: anomaly indicator
- 128: set of reconstruction parameters
- 130: look up table or database
- 132: reconstruction algorithm
- 134: synthesized magnetic resonance image
- 200: receive a set of magnetic resonance images descriptive of a field of view of a subject acquired according to a synthetic magnetic resonance imaging protocol
- 202: receive an anomaly indicator from an anomaly detection module in response to inputting at least one of the set of magnetic resonance images into the anomaly detection module
- 204: determine a set of reconstruction parameters using the anomaly indicator
- 206: reconstruct a synthesized magnetic resonance image from the set of magnetic resonance images and the set of reconstruction parameters
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: pulse sequence commands
- 332: k-space data
- 334: preliminary set of reconstruction parameters
- 336: trial magnetic resonance image
- 338: anomalous location
- 340: calculated image contrast range
- 342: predetermined image contrast range
- 400: acquire the k-space data by controlling the magnetic resonance imaging system with the pulse sequence commands
- 402: reconstruct the set of magnetic resonance images from the k-space data
- 404: set preliminary reconstruction parameters
- 406: reconstruct trial magnetic resonance image using the preliminary reconstruction parameters
- 408: Is image contrast within the anomaly location(s) within the predetermined image contrast range?
- 410: provide the trial magnetic resonance image as the synthesized magnetic resonance image
- 412: modify preliminary reconstruction parameters
- 600: graphical user interface
- 602: composite image

## Claims

1. A medical system (100, 300) comprising:
- a memory (110) storing machine executable instructions (120) and an anomaly detection module (122); and
- a computational system (104), wherein execution of the machine executable instructions causes the computational system to:
- receive (200) a set of magnetic resonance images (124) descriptive of a field of view (309) of a subject (318) acquired according to a synthetic magnetic resonance imaging protocol;
- receive (202) an anomaly indicator (126) from the anomaly detection module in response to inputting at least one of the set of magnetic resonance images into the anomaly detection module;
- determine (204) a set of reconstruction parameters (128) using the anomaly indicator; and
- reconstruct (206) a synthesized magnetic resonance image (134) from the set of magnetic resonance images and the set of reconstruction parameters.

2. The medical system of claim 1, wherein the field of view is descriptive of a right hemisphere and a left hemisphere of a brain of the subject, wherein the anomaly indicator comprises one or more anomalous locations in the field of view, and wherein the algorithmic anomaly detection module is further configured to spatially locate the one or more anomaly in the brain of the subject using a symmetrified contrast between the right hemisphere and the left hemisphere of the brain of the subject.

3. The medical system of claim 1, wherein the anomaly indicator comprises one or more anomalous locations in the field of view, wherein the anomaly detection module comprises an autoencoder neural network configured to output an autoencoded image for each of the at least one of the set of magnetic resonance images, wherein receiving an anomaly indicator in response to inputting the at least one of the set of magnetic resonance images into the anomaly detection module comprises:
- receiving the autoencoded image in response to inputting the at least one of the set of magnetic resonance images into the autoencoder neural network;
- determine the one or more anomalous locations by comparing the autoencoded image of each of the at least one of the set of magnetic resonance images with the at least one of the set of magnetic resonance images.

4. The medical system of claims 2 or 3, wherein execution of the machine executable instructions further causes the computational system to iteratively (406, 408, 412) vary the set of reconstruction parameters and reconstruct the synthesized magnetic resonance image to adjust the image contrast of the one or more anomalous locations to have a predetermined image contrast range (342).

5. The medical system of any one of claims 2 through 4, wherein the anomaly indicator indicates multiple anomalous locations, wherein the synthesized magnetic resonance image is reconstructed for each of the multiple anomalous locations resulting in a set of synthesized magnetic resonance images.

6. The medical system of claim 5, wherein execution of the machine executable instructions further causes the computational system to construct a composite image (602) from the set of synthesized magnetic resonance images locations by:
- including the anomalous location from each of the set of synthesized magnetic resonance images; and
- blending pixel values between the anomalous location from each of the set of synthesized magnetic resonance images using the set of synthesized magnetic resonance images.

7. The medical system of claim 6, wherein execution of the machine executable instructions further causes the computational system to:
- display the composite image on a graphical user interface (600);
- receive a selection of an anomalous location within the composite image from the graphical user interface; and
- display the synthesized magnetic resonance image comprising the selected anomalous location.

8. The medical system of claim 1, wherein the anatomical detection module comprises a convolutional neural network configured to output an anomaly classification as the anomaly indicator in response to receiving the at least one of the set of magnetic resonance images.

9. The medical system of claim 1, wherein the anatomical detection module comprises a variational autoencoder neural network that has a latent space, wherein the anomaly detection module is configured to output an anomaly classification as the anomaly indicator, wherein receiving an anomaly indicator in response to inputting the at least one of the set of magnetic resonance images into the anomaly detection module comprises:
- inputting the at least one the set of magnetic resonance images into the variational autoencoder; and
- determining the anomaly classification using an out of distribution detection method on the latent space of the variational autoencoder.

10. The medical system of claim 8 or 9, wherein the reconstruction parameters are determined by using the anomaly classification to search a reconstruction parameter look up table or a reconstruction parameter database.

11. The medical system of any one of the preceding claims, wherein reconstructing the synthesized magnetic resonance image comprises:
- determining a T1 dependent value, a T2 dependent value, and a proton density for each voxel of the field of view by performing a voxel wise fit of a chosen signal intensity equation to the set of magnetic resonance images; and
- reconstruct the synthesized magnetic resonance image by calculating a signal intensity value for each voxel using a reconstruction signal intensity equation that takes the voxel wise T1 dependent value, the voxel wise T2 dependent value, the voxel wise proton density value, and the reconstruction parameters as input.

12. The medical system of any one of claims 1 through 10, wherein the set of magnetic resonance images forms a magnetic resonance fingerprint, and wherein the synthesized magnetic resonance image is reconstructed from the set of magnetic resonance image according to a magnetic resonance fingerprinting protocol.

13. The medical system of any one of the preceding claims, wherein the medical system further comprises a magnetic resonance imaging system (302), where the memory further contains pulse sequence commands (330) configured to control the magnetic resonance imaging system to acquire k-space data (332) according to the synthetic magnetic resonance imaging protocol, wherein the execution of the machine executable instructions further causes the computational system to:
- acquire (400) the k-space data by controlling the magnetic resonance imaging system with the pulse sequence commands; and
- reconstruct (402) the set of magnetic resonance images from the k-space data.

14. A method of operating a medical system (100, 200), wherein the method comprises:
- receiving (200) a set of magnetic resonance images (124) descriptive of a field of view (309) of a subject (318) acquired according to a synthetic magnetic resonance imaging protocol;
- receiving (202) an anomaly indicator (126) from an anomaly detection module (122) in response to inputting at least one of the set of magnetic resonance images into the anomaly detection module;
- determining (204) a set of reconstruction parameters (128) using the anomaly indicator; and
- reconstructing (206) a synthesized magnetic resonance image (134) from the set of magnetic resonance images and the set of reconstruction parameters.

15. A computer program comprising machine executable instructions (120) for execution by a computational system (104), wherein the computer program further comprises an anatomical detection module (122) for execution by the computational system,
wherein execution of the machine executable instructions causes the computational system to:
- receive (200) a set of magnetic resonance images (124) descriptive of a field of view (309) of a subject (318) acquired according to a synthetic magnetic resonance imaging protocol;
- receive (202) an anomaly indicator (126) from an anomaly detection module in response to inputting at least one of the set of magnetic resonance images into the anomaly detection module;
- determine (204) a set of reconstruction parameters (128) using the anomaly indicator; and
- reconstruct (206) a synthesized magnetic resonance image (134) from the set of magnetic resonance images and the set of reconstruction parameters.
